# EUROPEAN PATENT APPLICATION

(11) **EP 4 806 406 A1**
(43) Date of publication of application: **16.09.2026**
(21) Application number: 24897488.3
(22) Date of filing: 25.11.2024
(51) Int. Cl.: A61F 13/15

(54) **MANUFACTURING DEVICE AND MANUFACTURING METHOD FOR ABSORBENT ARTICLE**

(30) Priority: 30.11.2023 JP 2023202685
(71) Applicant: Zuiko Corporation, Ibaraki-shi Osaka 5670082 (JP)
(72) Inventor: IWAMURA, Yosuke, Ibaraki-shi, Osaka 567-0082 (JP)
(74) Representative: SSM Sandmair
(86) International application number: PCT/JP2024/041625
(87) International publication number: WO 2025/115803

(57) **Abstract**

There are provided an apparatus and a method for manufacturing an absorbent article in which a problem with a continuous web for forming the absorbent article not being cut at a desired position can be detected at an early stage. A manufacturing apparatus (100) for a diaper (1) includes a first drum (200), a second drum (300), a cutting device (500), and a control device (700). The first drum conveys a continuous web. The second drum receives the continuous web conveyed by the first drum and further conveys the continuous web. The cutting device is disposed to face the second drum, and cuts a predetermined portion of the continuous web to form the diaper. The control device detects, on an upstream side of a cutting location at which the continuous web is to be cut by the cutting device in a conveyance direction of the continuous web, a portion to be cut of the continuous web that the first drum or the second drum conveys.

## Description

### Technical Field

The present invention relates to an apparatus and a method for manufacturing an absorbent article.

### Background Art

As disclosed in Patent Literature 1 (WO 2018/212092 A), an apparatus and a method for manufacturing an absorbent article are known in which a continuous web having stretchability in a conveyance direction is conveyed while tension is applied to the continuous web in the conveyance direction, and in which the conveyed continuous web is cut, thereby forming individual absorbent articles.

In particular, in the manufacturing apparatus disclosed in Patent Literature 1 (WO 2018/212092 A), by changing the tension applied to the conveyed continuous web, an amount of stretch of the continuous web is adjusted, which allows the size change of a product, and thus operations required at the time of the size change of the product can be reduced.

### Citation List

### Patent Literature

Patent Literature 1: WO 2018/212092 A

### Summary of Invention

### Technical Problem

However, in the case of using such a continuous web having stretchability, when the continuous web stretches or contracts with respect to a desired length for some reason, in a cutting step of cutting the continuous web to a length corresponding to the size of the absorbent article, there is a possibility that the continuous web is cut at a position shifted from a to-be-cut portion and that a product having a desired shape may not be obtained. There is a possibility that the occurrence of such a problem is not immediately detectable when the problem occurs.

### Solutions to Problem

An apparatus for manufacturing an absorbent article according to an embodiment of the present invention includes a first drum, a second drum, a cutting device, and a detection device. The first drum is configured to convey a continuous web. The second drum is configured to receive the continuous web conveyed by the first drum and further convey the continuous web. The cutting device is disposed to face the second drum, and is configured to cut a predetermined portion of the continuous web to form an absorbent article. The detection device is configured to detect, on an upstream side of a cutting location at which the continuous web is to be cut by the cutting device in a conveyance direction of the continuous web, a portion to be cut of the continuous web that the first drum or the second drum conveys.

A method for manufacturing an absorbent article according to an embodiment of the present invention includes a first conveyance step, a second conveyance step, a cutting step, and a detection step. In the first conveyance step, a continuous web is conveyed by a first drum. In the second conveyance step, the continuous web conveyed by the first drum is further conveyed by a second drum. In the cutting step, a predetermined portion of the continuous web is cut by a cutting device disposed to face the second drum to form an absorbent article. In the detection step, a portion to be cut of the continuous web that the first drum or the second drum conveys is detected by a detection device, on an upstream side of a cutting location at which the continuous web is to be cut by the cutting device in a conveyance direction of the continuous web.

### Advantageous Effects of Invention

In the apparatus and the method for manufacturing an absorbent article of the present invention, it is possible to detect a positional shift of a portion to be cut (a positional shift of a to-be-cut portion at a detection location or a positional shift of a portion to be actually cut by the cutting device) at an early stage, on the basis of an image.

### Brief Description of Drawings

Fig. 1 is a front view schematically depicting a disposable diaper as an example of an absorbent article manufactured by an apparatus for manufacturing an absorbent article of the present invention.
Fig. 2 is a view depicting the disposable diaper of Fig. 1 in a state where side sealed portions are opened.
Fig. 3 is a block diagram illustrating the apparatus for manufacturing an absorbent article according to an embodiment of the present invention, together with flows of steps.
Fig. 4A is a schematic process diagram illustrating some of steps of a method for manufacturing a wearing article of the embodiment.
Fig. 4B is a schematic process diagram illustrating some of the steps of the method for manufacturing a wearing article of the embodiment, the diagram illustrating a continuation of the steps illustrated in Fig. 4A.
Fig. 4C is a schematic process diagram illustrating some of the steps of the method for manufacturing a wearing article of the embodiment, the diagram illustrating a continuation of the steps illustrated in Fig. 4B.
Fig. 5 is a side view depicting the manufacturing apparatus of Fig. 3 partly (a first drum, a second drum, an imaging device, and a cutting device).
Fig. 6 is an enlarged view of the vicinity of the cutting device of Fig. 5.
Fig. 7 is an enlarged view of the cutting device, and secondary suction members and one of anvils of the second drum.
Fig. 8 includes views in which a view in an upper part is a view of the second drum (primary suction members and the secondary suction members of the second drum) as viewed along a radial direction of the second drum, and in which a view in a lower part is a view of a continuous web and the diapers conveyed by the second drum depicted in the upper part, as viewed along the radial direction of the second drum.

### Description of Embodiment

An embodiment of an apparatus and a method for manufacturing an absorbent article according to the present invention will be described below with reference to the drawings.

Note that the embodiment to be described below is simply an example of the present invention, and does not limit the scope of the present invention. It will be understood by a person skilled in the art that various changes can be made to the following embodiment without departing from the spirit and scope of the invention set forth in the claims.

### (1) Absorbent Article

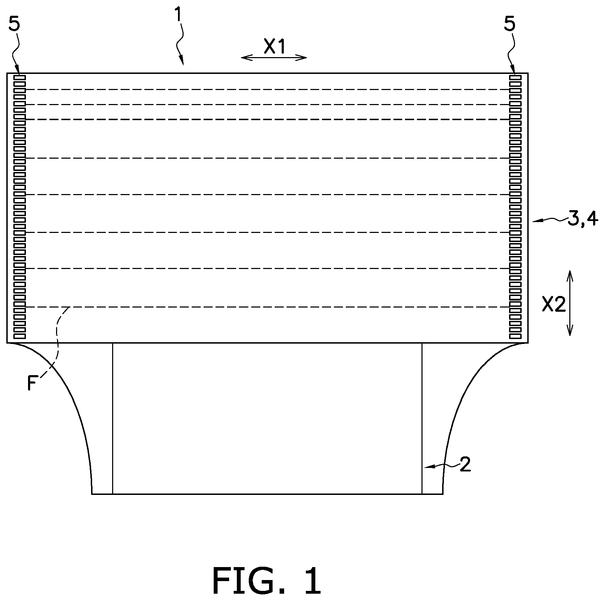

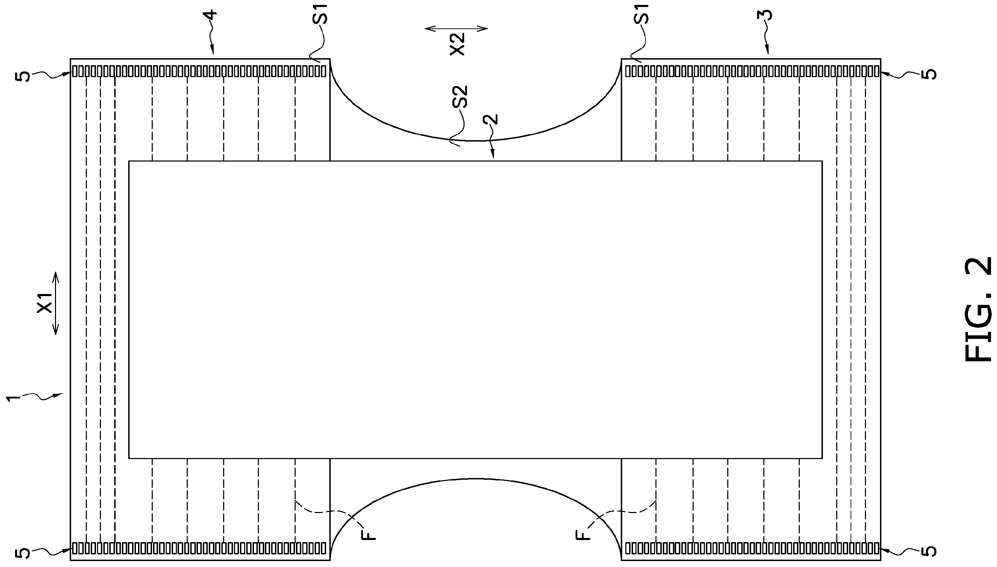

An example of an absorbent article manufactured by a manufacturing apparatus 100 according to an embodiment of the present invention will be described with reference to Figs. 1 and 2.

Fig. 1 is a front view schematically depicting a disposable diaper 1 (hereinafter, simply referred to as diaper 1) according to an example of the absorbent article manufactured by the manufacturing apparatus 100. Fig. 2 is a view depicting the diaper 1 in a state where side sealed portions 5 to be described later are opened.

In the present embodiment, the absorbent article will be described by taking the disposable diaper 1 as an example. However, the absorbent article is not limited to a disposable diaper, as long as the absorbent article is a wearing article including a member that absorbs liquid, such as an absorbent core to be described later, and formed by cutting a conveyed continuous web.

Details of the diaper 1 will be described.

Note that in the following description, expressions such as "front", "back", "left", "right" and the like may be used, and unless otherwise specified, these expressions respectively express "front", "back", "left", "right" and the like with reference to directions viewed from a wearer when the diaper 1 is worn by the wearer.

As illustrated in Fig. 1, the diaper 1 has a left-right symmetrical structure. As illustrated in Fig. 2, the diaper 1 mainly includes an absorbent body 2, a front waist portion 3, and a rear waist portion 4.

As illustrated in Fig. 2, the absorbent body 2 is a member that extends between the front waist portion 3 and the rear waist portion 4, and that covers the crotch of the wearer. The front waist portion 3 is a member that covers a front side of a torso of the wearer, and the rear waist portion 4 is a member that covers a back side of a torso of the wearer.

The absorbent body 2, the front waist portion 3, and the rear waist portion 4 forming the diaper 1 will be described in detail.

Note that in the following description, a direction extending around a waist of a wearer when worn by the wearer (left-right direction in Figs. 1 and 2) may be referred to as a waist width direction X1, and a direction orthogonal to the waist width direction X1, which is a longer direction of the absorbent body 2, may be referred to as a longitudinal direction X2.

Here, the diaper 1 is a pants-type diaper. However, the type of the diaper is not limited to the pants type, and may be a tape type in which a front waist portion and a rear waist portion are separated (not connected at the side sealed portion 5), and in which the front waist portion and the rear waist portion are fastened with a tape when the diaper is worn.

In the pants-type diaper 1 of the present embodiment, in the state of a finished article (the state illustrated in Fig. 1), the absorbent body 2 is folded in two at an intermediate portion in the longitudinal direction X2, and each of left and right end portions of the front waist portion 3 on both sides in the waist width direction X1 is overlapped with a corresponding one of left and right end portions of the rear waist portion 4 on both sides in the waist width direction X1 as illustrated in Fig. 1. An end portion of the front waist portion 3 in the waist width direction X1 and an end portion of the rear waist portion 4 in the waist width direction X1 are joined at the side sealed portion 5 (see Fig. 1).

The absorbent body 2 includes a top sheet, a back sheet, and an absorbent core arranged between the top sheet and the back sheet (illustration of the top sheet, the back sheet, and the absorbent core is omitted). Note that the structure of the absorbent body 2 illustrated here is simply an example, and the absorbent body 2 may include components other than the top sheet, the back sheet, and the absorbent core. The top sheet is a sheet that is arranged on a skin side of the wearer when worn, and is a liquid-permeable sheet. The back sheet is a sheet that is arranged on a side opposite to the top sheet (an outer side not facing the skin of the wearer), and is a liquid-impermeable sheet. The absorbent core is pulverized pulp or a mixture obtained by mixing the pulverized pulp with a superabsorbent polymer. The absorbent core absorbs bodily fluid such as urine of the wearer.

As illustrated in Fig. 2, the absorbent body 2 is a member having a substantially rectangular shape. The absorbent body 2 extends between the front waist portion 3 and the rear waist portion 4 with the longitudinal direction X2 as its longer direction. The absorbent body 2 is joined to the front waist portion 3 at a central portion of the front waist portion 3 in the waist width direction X1, and is joined to the rear waist portion 4 at a central portion of the rear waist portion 4 in the waist width direction X1.

The front waist portion 3 and the rear waist portion 4 include inner sheets S1 that face the skin of the wearer and an outer sheet S2 that does not face the skin of the wearer (arranged on an outer side when worn by the wearer). The inner sheet S1 and the outer sheet S2 are layered one on the other. Note that the outer sheet S2 not only forms the front waist portion 3 and the rear waist portion 4, but also continuously extends to connect the front waist portion 3 and the rear waist portion 4. The outer sheet S2 arranged between the front waist portion 3 and the rear waist portion 4 in the longitudinal direction X2 forms leg hole parts. In addition, as illustrated in Fig. 2, on the outer sheet S2 arranged between the front waist portion 3 and the rear waist portion 4 in the longitudinal direction X2, the absorbent body 2 is arranged.

In the front waist portion 3 and the rear waist portion 4, elastic members F are arranged in order to allow the diaper 1 to fit the wearer. In each of the front waist portion 3 and the rear waist portion 4, the elastic member F is arranged between the inner sheet S1 and the outer sheet S2, and in this state, the inner sheet S1 and the outer sheet S2 are joined. In addition, the elastic member F is joined to at least one of the inner sheet S1 and the outer sheet S2. As the elastic member F, for example, a plurality of rubber threads, flat elastic, or a material including thermoplastic resin can be adopted. Note that the structures of the front waist portion 3 and the rear waist portion 4 illustrated here are simply examples, and the front waist portion 3 and the rear waist portion 4 may include members other than the inner sheet S1, the outer sheet S2, and the elastic member F.

### (2) Outline of Apparatus and Method for Manufacturing Absorbent Article

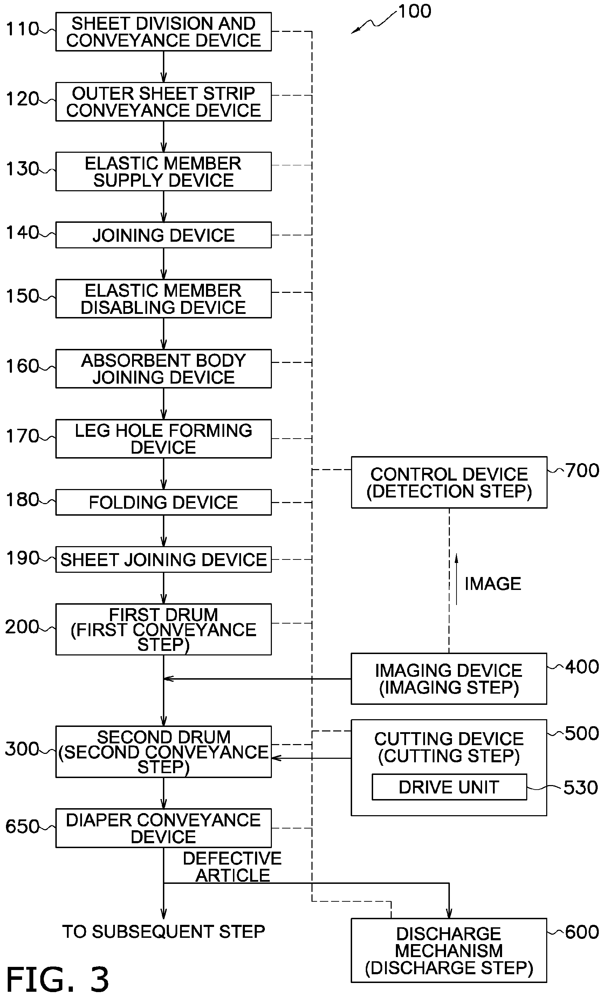

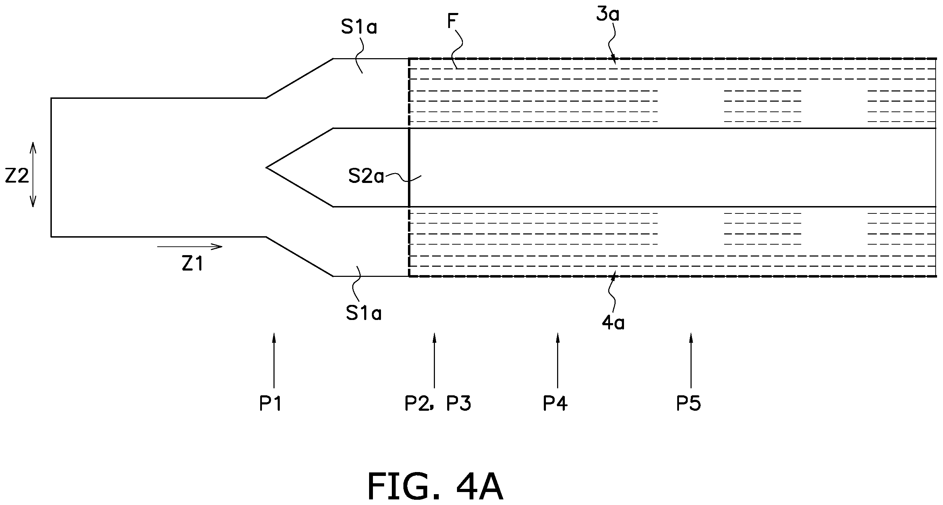

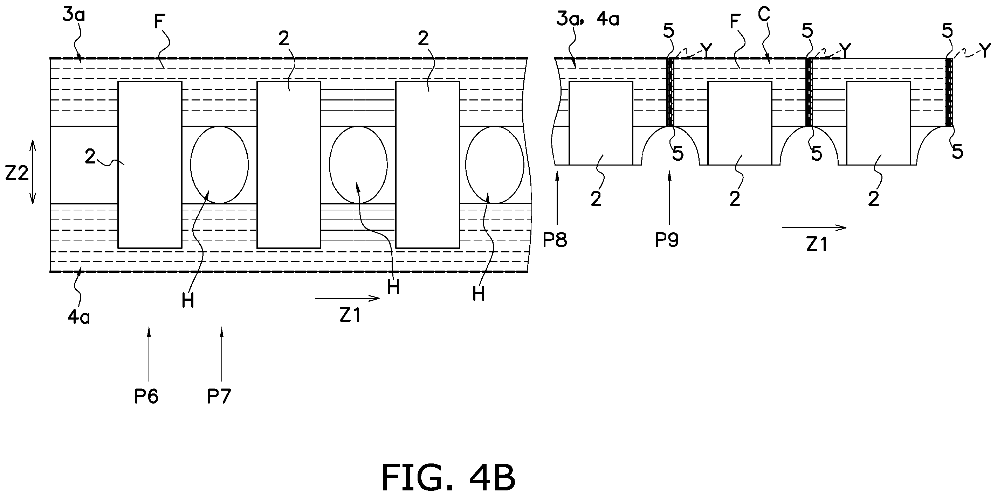

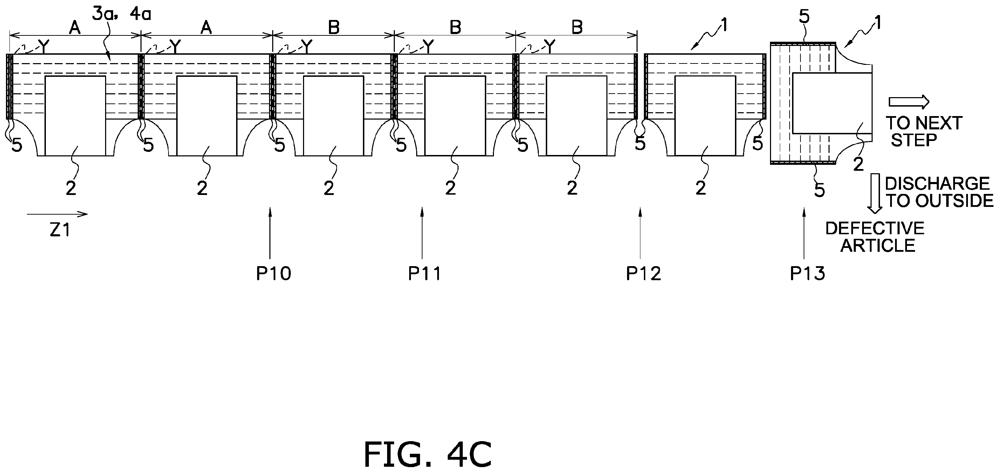

Outline of the manufacturing apparatus 100 and the method for manufacturing the diaper 1 will be described with reference to Figs. 3 and 4A to 4C. Fig. 3 is a block diagram illustrating the manufacturing apparatus 100 for the diaper 1, together with flows of steps. Figs. 4A to 4C are schematic process diagrams each illustrating some of steps of the method for manufacturing the diaper 1. Fig. 4B illustrates a continuation of the steps in Fig. 4A, and Fig. 4C illustrates a continuation of the steps in Fig. 4B. Note that the steps described below are simply examples, and may be changed as appropriate.

As illustrated in Fig. 3, the manufacturing apparatus 100 mainly includes a sheet division and conveyance device 110, an outer sheet strip conveyance device 120, an elastic member supply device 130, a joining device 140, an elastic member disabling device 150, an absorbent body joining device 160, a leg hole forming device 170, a folding device 180, a sheet joining device 190, a first drum 200, a second drum 300, an imaging device 400, a cutting device 500, a diaper conveyance device 650, a discharge device 600, and a control device 700.

The control device 700 is a computer including a CPU, memories such as a ROM and a RAM, an auxiliary storage device, an input/output device, various electric components and electronic components, and the like, illustration of which is omitted. Note that the control device 700 need not be a single device, and a plurality of devices may function as the control device 700 in cooperation.

The control device 700 is electrically connected to the sheet division and conveyance device 110, the outer sheet strip conveyance device 120, the elastic member supply device 130, the joining device 140, the elastic member disabling device 150, the absorbent body joining device 160, the leg hole forming device 170, the folding device 180, the sheet joining device 190, the first drum 200, the second drum 300, the imaging device 400, the cutting device 500, the diaper conveyance device 650, and the discharge device 600. The control device 700 controls, by causing the CPU to execute a program stored in the memory, operations of the sheet division and conveyance device 110, the outer sheet strip conveyance device 120, the elastic member supply device 130, the joining device 140, the elastic member disabling device 150, the absorbent body joining device 160, the leg hole forming device 170, the folding device 180, the sheet joining device 190, the first drum 200, the second drum 300, the imaging device 400, the cutting device 500, the diaper conveyance device 650, and the discharge device 600. In addition, the control device 700 functions as a detection device in the claims.

Note that, here, a case in which the control device 700 is the computer will be described as an example, but the control device 700 may be implemented by hardware (various electric circuits, electronic circuits) or may be implemented by cooperation of software and hardware, as long as the control device 700 can cause the various devices 110,120,130,140,150,160,170,180,190,200,300,400,500,600,650 to perform operations like operations to be described below, or can execute processing like processing to be described below, for example.

The sheet division and conveyance device 110 divides a continuously conveyed sheet fed from a sheet roll (not illustrated) around which the sheet is wound into two in a direction orthogonal to a conveyance direction with a blade (not illustrated) to form sheet-shaped inner sheet strips S1a (step P1 in Fig. 4A). Hereinafter, the conveyance direction of a sheet is referred to as a "conveyance direction Z1". In addition, a direction orthogonal to the "conveyance direction Z1" is referred to as a "width direction Z2". The inner sheet strip S1a is a strip of a sheet in which the inner sheets S1 each of which forms the front waist portion 3 when the diaper 1 is formed are continuously connected in a longer direction of the strip, or a strip of a sheet in which the inner sheets S1 each of which forms the rear waist portion 4 when the diaper 1 is formed are continuously connected in a longer direction of the strip. The sheet division and conveyance device 110 conveys the two inner sheet strips S1a formed by dividing the one sheet, in the same conveyance direction Z1, in a state where the two inner sheet strips S1a are separated from each other by a predetermined interval in the width direction Z2. The sheet division and conveyance device 110 conveys the stretchable inner sheet strips S1a while applying predetermined tension thereto in the conveyance direction Z1.

The outer sheet strip conveyance device 120 conveys a sheet-shaped outer sheet strip S2a (continuous sheet that becomes the outer sheet S2 at the time of formation into the diaper 1) fed from a sheet roll (not illustrated) around which the sheet is wound, in the same direction (in the conveyance direction Z1) as the direction in which the two rows of the inner sheet strips S1a are conveyed (see step P2 in Fig. 4A). The outer sheet strip conveyance device 120 conveys the stretchable outer sheet strip S2a while applying predetermined tension thereto in the conveyance direction Z1.

Each of the two rows of the inner sheet strips S1a conveyed by the sheet division and conveyance device 110 is aligned such that one end portion (outer edge portion) of the inner sheet strip S1a in the width direction Z2 overlaps with a corresponding one of both end portions of the outer sheet strip S2a in the width direction Z2.

Note that in another mode, each of the two rows of the inner sheet strips S1a conveyed by the sheet division and conveyance device 110 may be aligned such that the end portion (outer edge portion) of the inner sheet strip S1a in the width direction Z2 is arranged on an inner side relative to an end portion (outer edge portion) of the outer sheet strip S2a in the width direction Z2. Moreover, the end portion of the outer sheet strip S2a in the width direction Z2 (a portion arranged on an outer side relative to the inner sheet strip S1a in width direction Z2) may be folded inward to overlap with the absorbent body 2, in order to form a structure for inhibiting leakage of bodily fluid and the like from the waist portion of the diaper 1, in a subsequent step. Detailed description of this mode will be omitted.

The elastic member supply device 130 supplies the elastic member F in a state of being stretched in a longer direction thereof, between the inner sheet strip S1a and the outer sheet strip S2a (see step P3 in Fig. 4A). The elastic member supply device 130 arranges the elastic member F between the inner sheet strip S1a and the outer sheet strip S2a along the conveyance direction Z1.

Note that, here, for convenience, description is given for the manufacturing apparatus 100 in the order of the outer sheet strip conveyance device 120 and the elastic member supply device 130. However, in practice, the elastic member F is first supplied to the inner sheet strip S1a or the outer sheet strip S2a, and then the other sheet strip S2a or S1a is supplied, whereby the elastic member F is in a state of being sandwiched between the inner sheet strip S1a and the outer sheet strip S2a.

The joining device 140 joins the elastic member F, the inner sheet strip S1a, and the outer sheet strip S2a at a predetermined position to form each of a continuous sheet 3a that becomes the front waist portion 3 at the time of formation into the diaper 1 and a continuous sheet 4a that becomes the rear waist portion 4 at the time of formation into the diaper 1 (see step P4 in Fig. 4A). For example, a means such as thermal welding, ultrasonic welding, or an adhesive is used for the joining, though the means is not limited thereto. The continuous sheet 3a is a strip of a sheet in which the front waist portions 3 are continuously connected in a longer direction of the sheet, and the continuous sheet 4a is a strip of a sheet in which the front waist portions 3 are continuously connected in a longer direction of the sheet.

The elastic member disabling device 150 performs disabling processing of cutting a part of the elastic member F at a portion, of each of the continuous sheets 3a, 4a, where the absorbent body 2 is to be arranged (see step P5 in Fig. 4A). For cutting the elastic member F, for example, a blade may be used, or thermal cutting may be used.

The absorbent body joining device 160 arranges the absorbent body 2 at portions where the elastic member disabling device 150 has disabled the elastic members F such that the absorbent body 2 straddles the continuous sheet 3a and the continuous sheet 4a, and joins each of the continuous sheet 3a and the continuous sheet 4a, and the absorbent body 2 (see step P6 in Fig. 4B). For example, a means such as thermal welding, ultrasonic welding, or an adhesive is used for the joining, though the means is not limited thereto.

The leg hole forming device 170 sandwiches the continuous sheets 3a, 4a and the absorbent body 2 conveyed and having been integrated by the absorbent body joining device 160, for example, between an anvil roll and a die cut roll to form an oval-shaped hole H that becomes a leg hole of the diaper 1, in the outer sheet strip S2a (see step P7 in Fig. 4B).

After the hole H is formed in the outer sheet strip S2a, the folding device 180 folds the absorbent body 2 at a central portion in the width direction Z2, and places the continuous sheet 3a and the continuous sheet 4a one over the other (see step P8 in Fig. 4B). Note that in Fig. 4B, for convenience of illustration, the sheet is drawn in a discontinuous manner around the time point at which the folding is performed by the folding device 180, but in practice, cutting of the sheet along the width direction Z2 is not performed at this time point and the sheet is continuous.

The sheet joining device 190 joins the continuous sheet 3a and the continuous sheet 4a along the width direction Z2, in the vicinities of positions that become positions of both end portions of the front waist portion 3 and both end portions of the rear waist portion 4 at the time of formation into the diaper 1, in a layered sheet-shaped article (integrated continuous sheet 3a, continuous sheet 4a, and outer sheet strip S2a) in which the continuous sheet 3a and the continuous sheet 4a conveyed in a state of having been folded by the folding device 180 are layered one on the other, thereby forming the side sealed portions 5 (the side sealed portions 5 of each diaper 1) (see step P9 in Fig. 4B). In Fig. 4B, regions in which the side sealed portions 5 are formed are indicated by hatching. For example, a means such as thermal welding or ultrasonic welding is used for the joining, though the means is not limited thereto. Hereinafter, the continuous sheet 3a, the continuous sheet 4a, and the outer sheet strip S2a to which the absorbent bodies 2 are attached at predetermined intervals and that are joined at the side sealed portions 5 are referred to as a continuous web C.

The cutting device 500 to be described later cuts the continuous web C at an intermediate position between two side sealed portions 5 provided adjacent to each other in the conveyance direction Z1 (arranged to be interposed between two absorbent bodies 2 adjacent to each other in the conveyance direction Z1). Hereinafter, in order to simplify the description, a portion of the continuous web C to be cut by the cutting device 500 is referred to as a to-be-cut portion Y. In Figs. 4B and 4C, the to-be-cut portions Y are drawn with dashed lines. Note that the dashed lines drawn in Figs. 4B and 4C are imaginary lines, and are not lines actually provided on the continuous web C.

The first drum 200 is a device that conveys the continuous web C by rotating about a rotation shaft.

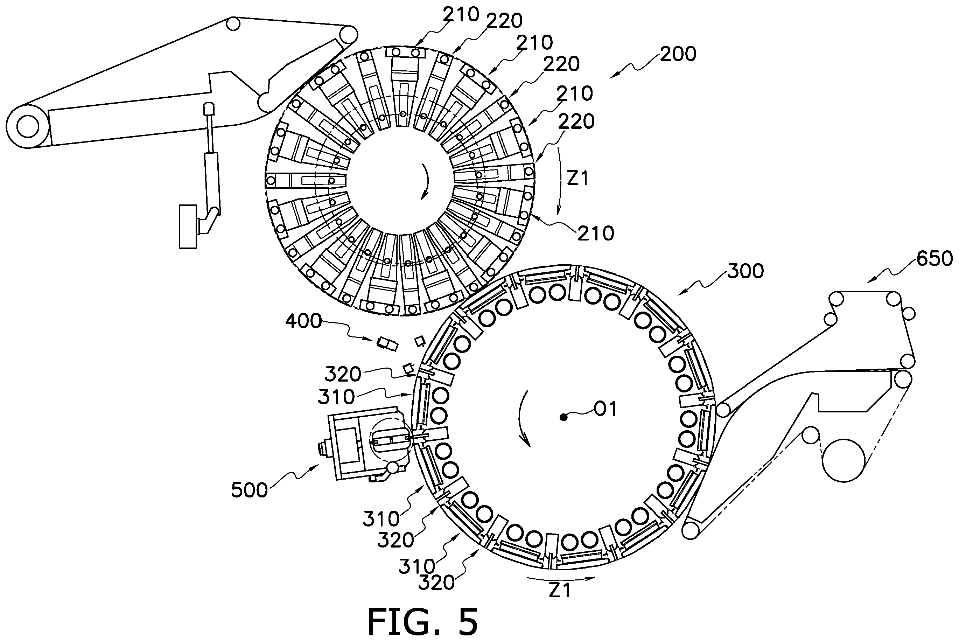

In particular, here, the first drum 200 is a sheet conveyance device including a plurality of suction members 210, 220 (see Fig. 5) each including a suction surface capable of sucking the continuous web C, a holding mechanism (not illustrated) that holds the plurality of suction members 210, 220 to be rotatable about a first axis, a drive device (not illustrated) including a rotation shaft (not illustrated) rotatable about a second axis parallel to the first axis and a connection mechanism (not illustrated) connecting the rotation shaft and the suction members 210, 220, and a support mechanism (not illustrated) supporting the holding mechanism and the drive device, in which the connection mechanism is connected to the plurality of suction members 210, 220 in a state where the plurality of suction members 210, 220 are movable in a radial direction of a circle about the first axis, and in which the support mechanism supports the holding mechanism and the drive device such that the holding mechanism and the drive device are relatively movable in a direction orthogonal to the first axis. Specifically, the first drum 200 is a device similar to a conveyance device for a sheet disclosed in Patent Literature 1 (WO 2018/212092 A).

The first drum 200 rotates in a direction of an arrow (clockwise in the case of Fig. 5) while sucking the continuous web C onto the suction surfaces (outer peripheral surfaces) of the suction members 210,220, thereby conveying the continuous web C in the conveyance direction Z1. The first drum 200 stretches and contracts the continuous web C while conveying the continuous web C such that the continuous web C can be cut at the same pitch (at intervals of the same length) by the cutting device 500, regardless of the size of the diaper 1, in a subsequent step. In other words, the first drum 200 adjusts the length, in the conveyance direction Z1, of the continuous web C that the first drum 200 conveys such that an interval between the to-be-cut portions Y in the continuous web C becomes a predetermined length regardless of the size of the diaper 1 to be manufactured. Further, in other words, the first drum 200 changes tension applied in the conveyance direction Z1 of the continuous web C, during the conveyance of the continuous web C. By using the first drum 200 capable of adjusting an amount of stretch of the continuous web C as described above, it is not necessary to perform an operation such as replacement of the second drum at the subsequent stage in accordance with the size of the diaper 1, even in a case where the size of the diaper 1 to be manufactured is changed (for example, even in a case where the diapers 1 of a medium size, a large size, and an extra-large size are manufactured by one manufacturing apparatus 100).

For example, as illustrated in Fig. 4C, the first drum 200 contracts the continuous web C in which a distance between the adjacent to-be-cut portions Y is A to cause the distance between the adjacent to-be-cut portions Y to be reduced to B (see step P10 in Fig. 4C).

The second drum 300 is a device that conveys the continuous web C by rotating about a rotation shaft. The second drum 300 has an outer shape that is a substantially circular shape as viewed along a rotation shaft O1 (rotation shaft that causes primary suction members 310 and secondary suction members 320, which will be described later, to rotate) (see Fig. 5).

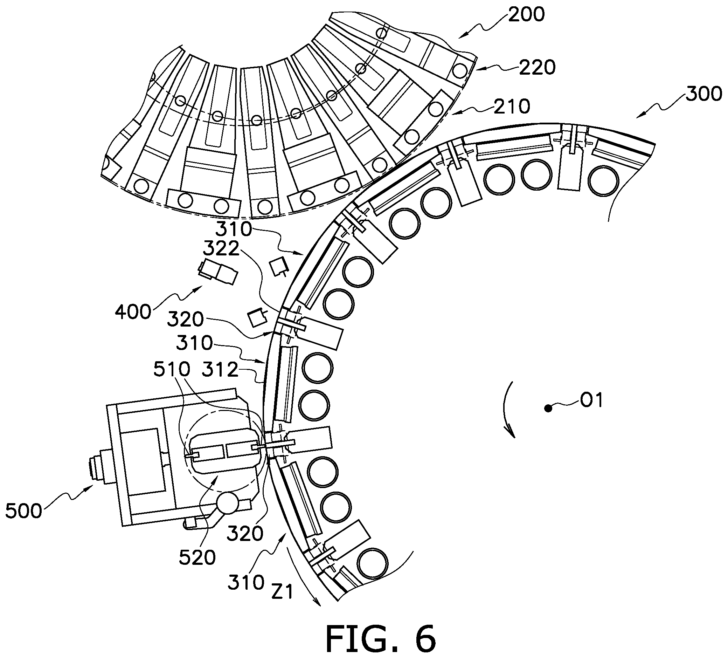

The second drum 300 receives the continuous web C (here, the continuous web C in which the distance between the adjacent to-be-cut portions Y is B) conveyed by the first drum 200, by using the primary suction members 310 and the secondary suction members 320 (see Fig. 5), and further conveys the continuous web C by causing the primary suction members 310 and the secondary suction members 320 to rotate about the rotation shaft O1, while sucking the continuous web C by using suction surfaces 312 (outer peripheral surfaces) of the primary suction members 310 and suction surfaces 322 (outer peripheral surfaces) of the secondary suction members 320 (see Fig. 6). Note that the continuous web C conveyed by the second drum 300 is cut by the cutting device 500, at the to-be-cut portion Y of the continuous web C, during the conveyance, and the diaper 1 that is the individual diaper 1 is formed (see step P12 in Fig. 4C). Note that, here, the expression that the diaper 1 is formed is not limited to a case in which the diaper 1 is in a state of being a finished article, and does not exclude, for example, a case in which some accessory is attached to the diaper 1 formed from the continuous web C or some processing is performed on the diaper 1, in a subsequent step.

The formed diaper 1 is further conveyed in the conveyance direction Z1 by the rotation of the primary suction member 310 and the secondary suction member 320 about the rotation shaft O1. Further, while conveying the diaper 1, the second drum 300 rotates the diaper 1, which has been conveyed in a state where the waist width direction X1 and the conveyance direction Z1 coincide with each other, by 90° to bring the diaper 1 into a state where the longitudinal direction X2 and the conveyance direction Z1 coincide with each other (see step P13 in Fig. 4C). Then, the second drum 300 further conveys, in the conveyance direction Z1, the diaper 1 in the state where the longitudinal direction X2 and the conveyance direction Z1 coincide with each other, and transfers the diaper 1 to the diaper conveyance device 650. The second drum 300 will be described in detail later.

The imaging device 400 is, for example, a camera that captures a moving image. In the conveyance direction Z1 of the continuous web C, at a location on an upstream side of a cutting location at which the continuous web C is to be cut by the cutting device 500 to be described later, the continuous web C is imaged (see step P11 in Fig. 4C). In the present embodiment, the control device 700 as an example of the detection device detects the portion to be cut (to-be-cut portion Y) of the continuous web C, on the basis of the image imaged by the imaging device 400. Note that the detection here is not limited to a case in which the control device 700 and the cutting device 500 detect the portion to be cut (to-be-cut portion Y) of the continuous web C itself, and may be detection in which the cutting device 500 detects a portion (for example, the side sealed portion 5 to be described later) on the basis of which the portion to be cut (to-be-cut portion) of the continuous web C can be identified. As illustrated in Fig. 5, the imaging device 400 preferably images the continuous web C that the second drum 300 conveys. For example, the imaging device 400 is installed at a position at which the imaging device 400 faces the second drum 300, with a lens facing the second drum 300, and images the continuous web C that the second drum 300 conveys.

In addition, the imaging device 400 may image the continuous web C that the first drum 200 conveys. For example, the imaging device 400 is installed at a position at which the imaging device 400 faces the first drum 200, with the lens facing the first drum 200, and images the continuous web C that the first drum 200 conveys. Note that in this case, the imaging device 400 preferably images the continuous web C after its tension has been changed in the first drum 200 (in the case of the present embodiment, after the distance between the adjacent to-be-cut portions Y has been changed to B).

The imaging device 400 preferably images at least a portion to be cut (to-be-cut portion Y) of the continuous web C by the cutting device 500. Specifically, for example, in the conveyance direction Z1 of the continuous web C, the imaging device 400 images the portion to be cut (to-be-cut portion Y) of the continuous web C by the cutting device 500 and the pair of side sealed portions 5 provided on opposite sides of the portion to be cut, in the continuous web C. Note that the imaging device 400 may image the continuous web C passing in front of the lens of the imaging device 400, in its entirety. Further, the imaging device 400 may image only a part of the side sealed portion 5, in the width direction Z2.

The cutting device 500 is a device that is disposed to face the second drum 300, and that cuts a predetermined portion (to-be-cut portion Y) of the continuous web C to form the diaper 1 as an example of the absorbent article.

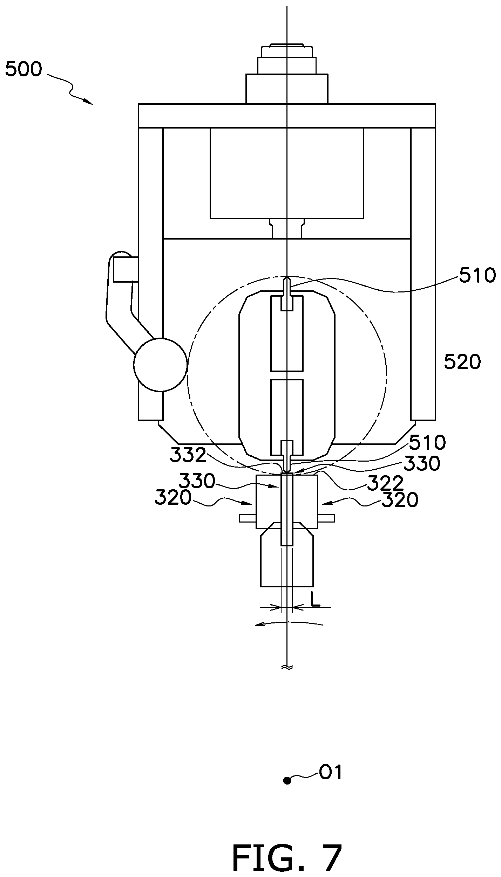

Although the structure of the cutting device 500 is not limited, the cutting device 500 includes, for example, one or more cutting members 510, a rotation mechanism 520 that causes the one or more cutting members 510 to rotate about a rotation shaft, and a drive unit 530 that drives the rotation mechanism 520 (see Figs. 3 and 6). The drive unit 530 is, for example, a motor, and drives the rotation mechanism 520 independently of the rotation of the second drum 300 (independently of a first drive unit 340 of the second drum 300 to be described later). When the cutting member 510 is caused to rotate by the rotation mechanism 520, the cutting member 510 moves between a position in contact with the continuous web C conveyed by the second drum 300 and a position not in contact with the continuous web C conveyed by the second drum 300. The control device 700 to be described later controls the operation of the drive unit 530 of the cutting device 500, whereby the control device 700 causes the cutting member 510 to come into contact with the continuous web C such that the continuous web C is sandwiched between the cutting member 510 and an anvil 330 (see Fig. 7), which will be described later, of the second drum 300, and causes the continuous web C to be cut. The control device 700 controls the operation of the cutting device 500, on the basis of the image imaged by the imaging device 400. The control on the operation of the cutting device 500 performed by the control device 700 will be described later.

Note that the cutting device 500 may be a device that causes the cutting member 510 to reciprocate such that the cutting member 510 approaches the second drum 300 and moves away from the second drum 300, instead of causing the cutting member 510 to rotate about the rotation shaft.

The diaper conveyance device 650 conveys the diaper 1 transferred from the second drum 300, to a subsequent step.

However, in a case where the diaper 1 is a defective article, the control device 700 causes the defective diaper 1 to be discharged to the outside (outside of the manufacturing process) by the discharge device 600 disposed downstream of the second drum 300, in the conveyance direction Z1 of the diaper 1. For example, in a case where it is determined that the continuous web C has not been cut at the to-be-cut portion Y by the cutting device 500, the control device 700 operates the discharge device 600 to remove the diaper 1 upstream and downstream of the position of cutting performed by the cutting device 500 from the diaper conveyance device 650. Note that the discharge device 600 may be, for example, a device that discharges the diaper 1 to the outside by causing a member to physically come into contact with the diaper 1, a device that discharges the diaper 1 to the outside by blowing air or the like onto the diaper 1, or a device that discharges the diaper 1 to the outside by guiding the defective diaper 1 to a path different from the path for the normal diaper 1.

Note that the manufacturing apparatus and the manufacturing process for the diaper 1 described here are simply examples, and can be appropriately changed.

For example, here, as an example, a case has been described in which the sheet division and conveyance device 110 divides the continuously conveyed sheet into two in the direction orthogonal to the conveyance direction with the blade (not illustrated) to form the sheet-shaped inner sheet strips S1a. However, without being limited to this, the inner sheet strip S1a for the continuous sheet 3a and the inner sheet strip S1a for the continuous sheet 4a may be fed from two respective separate sheet rolls. In addition, for example, the manufacturing process for the diaper 1 may include a step of attaching a member other than the members described above to the diaper 1 or a step of performing some processing on the diaper 1. Further, for example, the manufacturing apparatus 100 for the diaper 1 may include a device that provides notification of production of a defective article of the diaper 1, instead of including the discharge device 600 for the diaper 1.

### (3) Second Drum

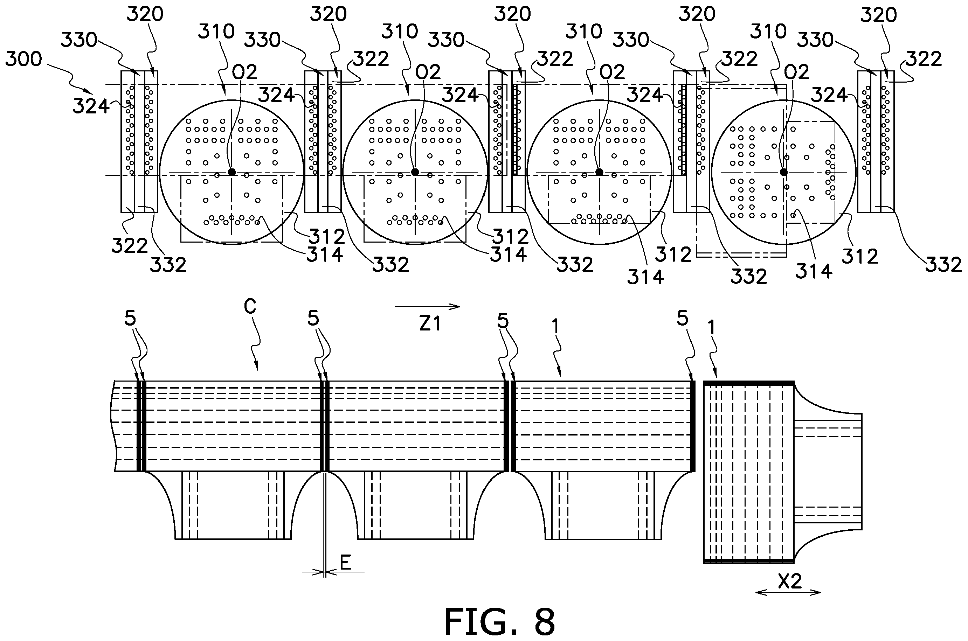

The second drum 300 will be described in detail with reference to Figs. 5 to 8 and the like. Fig. 5 is a side view depicting the manufacturing apparatus 100 partly (the first drum 200, the second drum 300, the imaging device 400, and the cutting device 500). Fig. 6 is an enlarged view of the vicinity of the cutting device 500 of Fig. 5. Fig. 7 is an enlarged view of the cutting device 500, and the secondary suction members 320 and one of anvils 330 of the second drum 300. Fig. 8 includes views in which a view in the upper part is a view of the second drum 300 (the primary suction members 310 and the secondary suction members 320 of the second drum 300) as viewed along a radial direction of the second drum 300 (a direction extending perpendicular to the rotation shaft O1 from the rotation shaft O1), and in which a view in the lower part is a view of the continuous web C and the diapers 1 conveyed by the second drum 300 depicted in the upper part, as viewed along the radial direction of the second drum 300.

The second drum 300 mainly includes the plurality of primary suction members 310, the plurality of secondary suction members 320, the plurality of anvils 330, the first drive unit 340 that causes the primary suction members 310, the secondary suction members 320, and the anvils 330 to rotate about the rotation shaft O1, and a second drive unit 350 that causes the primary suction members 310 to rotate about rotation shafts O2 each extending in the radial direction of the second drum 300 (see Figs. 3, and 5 to 8).

The plurality of primary suction members 310 and the plurality of secondary suction members 320 are alternately arranged in a circumferential direction about the rotation shaft O1 (see Fig. 5). The plurality of anvils 330 are arranged in the circumferential direction about the rotation shaft O1 of the second drum 300. Specifically, each of the anvils 330 is disposed at a corresponding one of central portions of the respective secondary suction members 320, in the circumferential direction about the rotation shaft O1 (see Fig. 7). In other words, the anvils 330 are alternately arranged with the primary suction members 310 in the circumferential direction about the rotation shaft O1, and the secondary suction members 320 as an example of support portions that support the continuous web C are provided on both sides of the anvil 330 in the circumferential direction of the second drum 300 about the rotation shaft O1 (see Figs. 6 and 7).

### (3-1) Primary Suction Member and Secondary Suction Member

The primary suction member 310 includes the suction surface 312 on a side opposite to the rotation shaft O1 side (on the outer peripheral surface) (see Figs. 6 and 8). A plurality of suction holes 314 that suck air are formed in the suction surface 312 (see Fig. 8).

The secondary suction member 320 includes the suction surface 322 on a side opposite to the rotation shaft O1 side (on the outer peripheral surface) (see Figs. 6 and 8). A plurality of suction holes 324 that suck air are formed in the suction surface 322 (see Fig. 8).

The primary suction member 310 sucks and holds the continuous web C transferred from the first drum 200 to the suction surface 312 by a suction device (not illustrated), disposed inside the second drum 300, sucking air from the suction holes 314 of the suction surface 312. The secondary suction member 320 sucks and holds the continuous web C transferred from the first drum 200 to the suction surface 322 by a suction device (not illustrated), disposed inside the second drum 300, sucking air from the suction holes 324 of the suction surface 322. Specifically, the suction surface 312 of the primary suction member 310 sucks and holds the absorbent body 2 and a portion to which the absorbent body 2 is attached and its periphery of each of the continuous sheet 3a and the continuous sheet 4a of the continuous web C (see Fig. 8). In other words, the suction surface 312 of the primary suction member 310 sucks and holds a central portion, in the waist width direction X1, of the diaper 1 to be formed from the continuous web C. The suction surface 322 of the secondary suction member 320 sucks and holds the vicinity of the to-be-cut portion Y of the continuous web C (see Fig. 8). In other words, the suction surface 322 of the secondary suction member 320 sucks and holds an end portion, in the waist width direction X1, of the diaper 1 to be formed from the continuous web C.

### (3-2) Anvil

The anvil 330 is a member that serves as a receiving member for the cutting member 510 and that sandwiches the continuous web C (in particular, the to-be-cut portion Y of the continuous sheets 3a, 4a) between an outer peripheral surface 332 of the anvil 330 and the cutting member 510 of the cutting device 500 in a cutting step for the continuous web C performed by the cutting device 500.

The outer peripheral surface 332 of the anvil 330 is preferably disposed on an outer side relative to the suction surface 312 of the primary suction member 310 and the suction surface 322 of the secondary suction member 320 (on a side where the cutting device 500 is disposed with respect to the second drum 300) in a radial direction about the rotation shaft O1 such that the continuous web C is easily sandwiched between the outer peripheral surface 332 and the cutting member 510 (see Fig. 7).

In addition, for the anvil 330, a width L of the anvil 330 (see Fig. 7) is preferably longer than a width E between the side sealed portions 5, adjacent to each other, of the continuous web C conveyed on the second drum 300 (a distance between the pair of side sealed portions 5 adjacently arranged on opposite sides of one to-be-cut portion Y, see Fig. 8), in the circumferential direction about the rotation shaft O1 (the conveyance direction Z1 of the continuous web C). With such a configuration, even if the center of the outer peripheral surface 332 of the anvil 330 in the circumferential direction about the rotation shaft O1 and a position of the to-be-cut portion Y of the continuous web C are slightly shifted, the to-be-cut portion Y of the continuous web can be sandwiched between the cutting member 510 and the anvil 330 when the continuous web C is cut by the cutting device 500.

### (3-3) First Drive Unit and Second Drive Unit

The first drive unit 340 causes the continuous web C to be conveyed in the conveyance direction Z1 by causing the primary suction member 310 and the secondary suction member 320 sucking and holding the continuous web C to rotate about the rotation shaft O1.

Note that when the secondary suction member 320 (the continuous web C sucked and held by the secondary suction member 320) reaches a location facing the cutting device 500, the cutting member 510 is caused to rotate by the rotation mechanism 520 of the drive unit 530 of the cutting device 500, and the cutting member 510 is caused to protrude toward the continuous web C. Then, the cutting member 510 of the cutting device 500 sandwiches the continuous web C (more specifically, the to-be-cut portion Y of the continuous web C) between the cutting member 510 and the anvil 330, and cuts and separates the diaper 1 from the continuous web C placed on an upstream side in the conveyance direction Z1, thereby forming the diaper 1. Note that how the control device 700 drives the drive unit 530 of the cutting device 500 such that the cutting member 510 comes into contact with the to-be-cut portion Y of the continuous web C will be described later.

As well as the continuous web C, the first drive unit 340 causes the diaper 1 to be conveyed in the conveyance direction Z1 by causing the primary suction member 310 and the secondary suction member 320 sucking and holding the diaper 1, cut by the cutting device 500 to be formed as the diaper 1, to rotate about the rotation shaft O1.

Note that when the diaper 1 is caused to be conveyed to a predetermined position by the first drive unit 340, the secondary suction member 320 supporting the end portion, in the waist width direction X1, of the diaper 1 stops the suction of the air from the suction holes 324 of the suction surface 322, and releases the suction and holding of the diaper 1. As a result, the diaper 1 is sucked and held only by the primary suction member 310. In this state, the second drive unit 350 causes the primary suction member 310 to rotate by 90° about the rotation shaft O2 (see Fig. 8) extending in the radial direction of the second drum 300, and causes the longitudinal direction X2 of the diaper 1 and the conveyance direction Z1 to coincide with each other.

In this conveyance state, the first drive unit 340 further causes the diaper 1 to be conveyed in the conveyance direction Z1 by causing the primary suction member 310 sucking and holding the diaper 1 to rotate about the rotation shaft O1. Then, at a timing when the diaper 1 is transferred from the second drum 300 to the diaper conveyance device 650, the primary suction member 310 stops the suction of the air from the suction holes 314 of the suction surface 312, and releases the suction and holding of the diaper 1.

### (4) Control on Cutting Device and Discharge Device

As described above, when the secondary suction member 320 (the continuous web C sucked and held by the secondary suction member 320) reaches the location facing the cutting device 500 (referred to as a cutting location), the cutting device 500 causes the cutting member 510 to protrude toward the continuous web C, sandwiches the to-be-cut portion Y of the continuous web C between the cutting member 510 and the anvil 330, and cuts and separates the diaper 1 from the continuous web C placed on the upstream side in the conveyance direction Z1, thereby forming the diaper 1.

Assuming that, as an ideal state, no positional shift occurs in the to-be-cut portion Y of the continuous web C, the to-be-cut portion Y of the continuous web C is designed to be constantly positioned at the center of the anvil 330 in the circumferential direction of the second drum 300 about the rotation shaft O1. In such a state, when the second drum 300 rotates at a constant speed, the continuous web C is expected to be always cut at the to-be-cut portion Y by constantly causing the cutting member 510 of the cutting device 500 to come into contact with the continuous web C at constant time intervals (time intervals in accordance with the rotation speed of the second drum 300).

However, in practice, for example, due to a deviation in an amount of stretch of the continuous web C in the first drum 200 or the like, the to-be-cut portion Y of the continuous web C may be shifted from the central position of the anvil 330, in the circumferential direction of the second drum 300 about the rotation shaft O1.

Therefore, here, the control device 700 detects the to-be-cut portion Y of the continuous web C, on the basis of the image imaged by the imaging device 400, and controls the operation of the cutting device 500 (a timing at which the cutting device 500 sandwiches the continuous web C between the cutting member 510 and the anvil 330 of the second drum 300), on the basis of the detection result.

Specifically, the imaging device 400 images the continuous web C immediately before being cut by the cutting device 500 (the continuous web C before being cut by the cutting device 500, conveyed by the second drum 300, or the continuous web C after being subjected to tension adjustment (after being subjected to adjustment of the amount of stretch) by the first drum 200, conveyed by the first drum 200). The control device 700 applies a typical image recognition technology to the image imaged by the imaging device 400, detects a portion, serving as a reference for a position, on the basis of which the to-be-cut portion Y can be identified, in the continuous web C, and grasps a timing at which the portion serving as a reference for a position has passed in front of the imaging device 400 (a timing (time) at which the portion serving as a reference for a position in the continuous web C has been imaged).

Further, the control device 700 grasps conveyance speeds of the continuous web C in the first drum 200 and the second drum 300, a distance in the conveyance direction Z1 between the portion serving as a reference for a position in the continuous web C and the to-be-cut portion Y, a distance in the conveyance direction Z1 from a location at which the continuous web C is imaged by the imaging device 400 to a cutting location at which cutting is to be performed by the cutting device 500, and the like. For example, these pieces of information are input to the control device 700 in advance. In addition, as long as a quantity is a quantity that can be detected by a sensor, such as each of the conveyance speeds of the continuous web C in the first drum 200 and the second drum 300, the control device 700 may acquire this quantity by using the sensor.

Then, from the timing (time) at which the portion serving as a reference for a position in the continuous web C has been imaged, the conveyance speeds of the continuous web C in the first drum 200 and the second drum 300, the distance in the conveyance direction Z1 between the portion serving as a reference for a position in the continuous web C and the to-be-cut portion Y, the distance in the conveyance direction Z1 from the location at which the continuous web C is imaged by the imaging device 400 to the cutting location at which cutting is to be performed by the cutting device 500, and the like, the control device 700 calculates a timing (time) at which the to-be-cut portion Y of the continuous web C reaches the cutting location at which cutting is to be performed by the cutting device 500. The control device 700 controls the operation of the drive unit 530 of the cutting device 500 such that the cutting member 510 comes into contact with the continuous web C at this timing, thereby adjusting the rotation speed of the cutting member 510 through the rotation mechanism 520. As a result of such a configuration, even if the to-be-cut portion Y of the continuous web C is shifted from the central position of the anvil 330 in the circumferential direction of the second drum 300 about the rotation shaft O1 due to a deviation in the amount of stretch of the continuous web C in the first drum 200 or the like, the cutting device 500 can cut the continuous web C at the to-be-cut portion Y or in the immediate vicinity of the to-be-cut portion Y (at a position at which the shift does not adversely affect the quality of the diaper 1).

Note that the portion serving as a reference for a position in the continuous web C is preferably, for example, the pair of side sealed portions 5 provided on opposite sides of the to-be-cut portion Y. The position of the side sealed portion 5 is close to the position of the to-be-cut portion Y, and thus the continuous web C can be accurately cut at an appropriate position by using the side sealed portion 5 as the portion serving as a reference for a position in the continuous web C.

However, the portion serving as a reference for a position in the continuous web C is not limited to the side sealed portion 5, and may be, for example, a design or the like provided on the continuous sheet 3a or the continuous sheet 4a, or may be an edge portion of the absorbent body 2 in the conveyance direction Z1. In addition, in a case where the diaper is not a pants-type diaper but a tape-type diaper, a fastening tape affixed on the continuous web C may be used as the portion serving as a reference for a position in the continuous web C.

Further, the control device 700 may detect the to-be-cut portion Y of the continuous web C itself, as the portion, serving as a reference for a position, on the basis of which the to-be-cut portion Y can be identified, in the continuous web C.

Note that, in such a case where, for some reason, the adjustment of the rotation speed through the rotation mechanism 520 fails to handle the positional shift of the to-be-cut portion Y and the cutting of the continuous web C at the to-be-cut portion Y or in its vicinity fails, the control device 700 causes the defective diaper 1 to be discharged to the outside (outside of the manufacturing process) by the discharge device 600 disposed downstream of the second drum 300 in the conveyance direction Z1 of the diaper 1.

### (5) Characteristics

(5-1)
The manufacturing apparatus 100 for the diaper 1 as an example of an absorbent article includes the first drum 200, the second drum 300, the cutting device 500, and the control device 700 as an example of the detection device. The first drum 200 conveys the continuous web C. The second drum 300 receives the continuous web C conveyed by the first drum 200 and further conveys the continuous web C. The cutting device 500 is disposed to face the second drum 300, and cuts a predetermined portion of the continuous web C to form the diaper 1. The control device 700 detects, on the upstream side of the cutting location at which the continuous web C is to be cut by the cutting device 500 in the conveyance direction of the continuous web C, the portion to be cut (to-be-cut portion Y) of the continuous web C that the first drum 200 or the second drum 300 conveys.

In the manufacturing apparatus 100, it is possible to detect a positional shift of the portion to be cut (a positional shift of the to-be-cut portion Y at the detection location or a positional shift of a portion to be actually cut by the cutting device 500) at an early stage.

(5-2)
The manufacturing apparatus 100 for the diaper 1 includes the imaging device 400 that images the continuous web C. The control device 700 detects the portion to be cut (to-be-cut portion Y) of the continuous web C, on the basis of the image imaged by the imaging device 400.

In this manufacturing apparatus, it is possible to detect the positional shift of the portion to be cut at an early stage, on the basis of the image.

(5-3)
The manufacturing apparatus 100 for the diaper 1 includes the control device 700 as an example of a first controller that controls the operation of the cutting device 500, on the basis of detection performed by the control device 700.

Note that, here, the control device 700 functions as the detection device and the first controller, but, without being limited thereto, a first device may function as the detection device, and a second device different from the first device may function as the first controller.

In the manufacturing apparatus 100, it is possible to reduce the positional shift of the portion to be cut of the continuous web C.

For example, preferably, the manufacturing apparatus 100 for the diaper 1 includes the imaging device 400, and the imaging device 400 images the side sealed portions 5 as an example of a pair of sealed portions provided in the continuous web C and on opposite sides of the portion to be cut (to-be-cut portion Y), in the conveyance direction Z1 of the continuous web C. The control device 700 detects the portion to be cut (to-be-cut portion Y) from the detection result for the side sealed portions 5. The control device 700 controls the operation of the cutting device 500, on the basis of a timing at which the side sealed portions 5 are imaged.

In such a manufacturing apparatus for the diaper 1, the side sealed portion 5, which is relatively easily detected, is used as a reference for detecting the positional shift of the portion to be cut (to-be-cut portion Y). Thus, it is possible to accurately detect the positional shift of the portion to be cut (to-be-cut portion Y) of the continuous web C at the imaging location, and therefore, it is possible to accurately control the portion to be cut of the continuous web C.

In addition, the side sealed portion 5 is close in distance from the to-be-cut portion Y. Thus, by using the side sealed portion 5 as a reference, it is possible to detect the positional shift of the portion to be cut (to-be-cut portion Y) of the continuous web C at the imaging location with particularly excellent accuracy, and therefore, it is possible to accurately control the portion to be cut of the continuous web C.

(5-4)
In the manufacturing apparatus 100 for the diaper 1, the second drum 300 includes the plurality of anvils 330 arranged in the circumferential direction of the second drum 300. The cutting device 500 includes the cutting member 510. The cutting device 500 cuts the continuous web C by sandwiching the continuous web C between the cutting member 510 and the anvil 330 of the second drum 300. The width L of each anvil 330 in the circumferential direction of the second drum 300 (the circumferential direction about the rotation shaft O1) is wider than the width E between the pair of side sealed portions 5 provided in the continuous web C and on opposite sides of the to-be-cut portion Y.

In the manufacturing apparatus 100, by setting the width L of the anvil 330 to be wider than the width E of the pair of side sealed portions 5 provided in the continuous web C and on opposite sides of the portion to be cut (to-be-cut portion Y), it is possible to perform the cutting while sandwiching the to-be-cut portion Y between the cutting member 510 and the anvil 330 even when the cutting timing of the cutting device 500 is slightly shifted in accordance with the positional shift.

(5-5)
In the manufacturing apparatus 100 for diaper 1 of the present embodiment, the second drum 300 includes the secondary suction members 320 as an example of the support portions that are provided on both sides of each of the anvils 330 in the circumferential direction about the rotation shaft O1 of the second drum 300 and that support the continuous web C.

In particular, here, the secondary suction member 320 not only simply supports the continuous web C but also sucks and holds the continuous web C.

In the manufacturing apparatus 100, the continuous web C is supported by the secondary suction members 320 provided on both sides of the anvil 330 in the circumferential direction about the rotation shaft O1 of the second drum 300. Thus, it is possible to reduce the occurrence of a problem such as a positional shift due to the occurrence of wrinkles in the continuous web C at the time of cutting.

(5-6)
The manufacturing apparatus 100 for the diaper 1 of the present embodiment includes the discharge device 600. The discharge device 600 is disposed downstream of the second drum 300, in the conveyance direction of the continuous web C, and discharges the diaper 1 to the outside (to the outside of the manufacturing process performed by the manufacturing apparatus 100). The control device 700 as an example of a second controller controls the operation of the discharge device 600. The control device 700 causes, by controlling the operation of the discharge device 600, the defective diaper 1 (for example, the diaper 1 that has not been cut at the to-be-cut portion Y) to be discharged to the outside.

Note that, here, the control device 700 functions as the detection device, the first controller, and the second controller, but, without being limited thereto, for example, a first device may function as the detection device, a second device different from the first device may function as the first controller, and a third device different from the first and second devices may function as the second controller.

In this manufacturing apparatus 100, even when the continuous web C is not cut at an appropriate position, it is possible to automatically discharge the generated defective diaper 1 to the outside.

(5-7)
In the manufacturing apparatus 100 for the diaper 1 of the present embodiment, the first drum 200 changes the tension applied to the continuous web C in the conveyance direction Z1 of the continuous web C, during conveyance of the continuous web C. The control device 700 detects the portion to be cut (to-be-cut portion Y) of the continuous web C after the tension has been changed in the first drum 200.

In the manufacturing apparatus 100 of the present embodiment, by performing tension adjustment in the first drum 200, the diapers 1 having different sizes can be manufactured by one apparatus, without performing complicated adjustment or the like. On the other hand, in a case where there are no measures, the continuous web C stretches and contracts with respect to a desired length when tension adjustment is performed, and in the cutting step for the continuous web C, there is a possibility that the continuous web C is cut at a position shifted from the to-be-cut portion Y and that a desired product may not be obtained. However, here, the control device 700 functions as the detection device, whereby it is possible to detect the positional shift of the to-be-cut portion Y at the detection location or the positional shift of the portion to be actually cut by the cutting device 500. Thus, it is possible to detect the occurrence of such a problem, and reduce the occurrence of such a problem, for example.

### (6) Modifications

Modifications of the embodiment described above will be described below. Note that the following modifications may be appropriately combined as long as they do not contradict each other.

### (6-1) Modification A

In the embodiment described above, as an example, a case is described in which the first drum 200 changes tension applied in the conveyance direction Z1 of the continuous web C, during the conveyance of the continuous web C. However, without being limited to this, the first drum 200 that supplies the continuous web C to the second drum 300 may be a conveyance device that does not perform such tension change (adjustment of the amount of stretch).

Even in such a case, and in a case where the positional shift of the to-be-cut portion Y of the continuous web C having stretchability in the conveyance direction Z1 can occur for some reason, it is useful to control the operation of the cutting device 500 using the detection device that detects the portion to be cut of the continuous web C.

### (6-2) Modification B

In the embodiment described above, as an example, a case is described in which the detection result of the control device 700 is used for controlling the operation of the cutting device 500. However, the detection result of the control device 700 may be used for a purpose other than the control on the operation of the cutting device 500.

For example, even in a case where the control device 700 constantly operates the cutting device 500 at constant time intervals (time intervals in accordance with the rotation speed of the second drum 300), when the control device 700 detects the positional shift of the to-be-cut portion Y in the continuous web C from the detection result of the control device 700 as the detection device, formation of the defective diaper 1 can be detected at an early stage.

In a case where the detection result of the control device 700 is used in such use, for example, the control device 700 may notify, by using some means, an administrator of the manufacturing apparatus 100 of the fact that the defective diaper 1 has been formed, or may stop the operation of the manufacturing apparatus 100.

### (6-3) Modification C

In the embodiment described above, as an example, the case is described in which the detection result of the control device 700 is used for controlling the operation of the cutting device 500. However, the detection result of the control device 700 may be used for controlling the operation of the second drum 300 (controlling the conveyance speed of the second drum 300).

However, the second drum 300 is typically large equipment, and thus, in many cases, it is preferable to control the operation of the cutting device 500 that is relatively small on the basis of the detection result of the control device 700, rather than to control the operation of the second drum 300 on the basis of the detection result of the control device 700.

### (6-3) Modification D

In the embodiment described above, the control device 700 as the detection device detects the portion to be cut (to-be-cut portion Y) of the continuous web C on the basis of the image imaged by the imaging device 400. However, the control device 700 as the detection device may detect the portion to be cut (to-be-cut portion Y) of the continuous web C by using other means.

For example, the manufacturing apparatus 100 for the diaper 1 may include a sensor that detects a thickness of the continuous web C, instead of the imaging device 400. In this case, the control device 700 as the detection device may detect, for example, the side sealed portion 5 having a thickness different from the thickness of another portion, on the basis of the detection result of the sensor, and detect the center between the two adjacent side sealed portions 5 as the portion to be cut (to-be-cut portion Y).

In addition, the side sealed portion 5 or the like may be detected by using and using ultraviolet rays, a change in reflected light in accordance with a material, or ultrasonic waves.

### <Additional Notes>

Finally, technical ideas that can be grasped from the embodiment described above will be additionally described below.

An apparatus for manufacturing an absorbent article according to a first aspect includes a first drum, a second drum, a cutting device, and a detection device. The first drum is configured to convey a continuous web. The second drum is configured to receive the continuous web conveyed by the first drum and further convey the continuous web. The cutting device is disposed to face the second drum, and is configured to cut a predetermined portion of the continuous web to form an absorbent article. The detection device is configured to detect, on an upstream side of a cutting location at which the continuous web is to be cut by the cutting device in a conveyance direction of the continuous web, a portion to be cut of the continuous web that the first drum or the second drum conveys.

In the manufacturing apparatus according to the first aspect, it is possible to detect a positional shift of the portion to be cut (a positional shift of a to-be-cut portion at a detection location or a positional shift of a portion to be actually cut by the cutting device) at an early stage.

An apparatus for manufacturing an absorbent article according to a second aspect is the apparatus for manufacturing an absorbent article according to the first aspect, further including an imaging device configured to image the continuous web. The detection device is configured to detect the portion to be cut of the continuous web, based on an image imaged by the imaging device.

In the manufacturing apparatus according to the second aspect, it is possible to detect the positional shift of the portion to be cut at an early stage, based on the image.

An apparatus for manufacturing an absorbent article according to a third aspect is the apparatus for manufacturing an absorbent article according to the second aspect, in which the imaging device is configured to image at least the portion to be cut of the continuous web by the cutting device.

An apparatus for manufacturing an absorbent article according to a fourth aspect is the apparatus for manufacturing an absorbent article according to any one of the first to third aspects, further including a first controller configured to control an operation of the cutting device, based on a detection result of the detection device.

In the manufacturing apparatus according to the fourth aspect, the positional shift of the portion to be cut (to-be-cut portion) of the continuous web at the detection location is detected based on the detection result of the detection device, based on which the operation of the cutting device is controlled. Thus, it is possible to reduce the positional shift of the portion to be cut of the continuous web.

An apparatus for manufacturing an absorbent article according to a fifth aspect is the apparatus for manufacturing an absorbent article according to the second or third aspect, further including a first controller configured to control an operation of the cutting device, based on a detection result of the detection device. The imaging device is configured to image a pair of sealed portions provided in the continuous web and on opposite sides of the portion to be cut, in the conveyance direction of the continuous web. The first controller is configured to control the operation of the cutting device, based on a timing at which the pair of sealed portions are imaged.

Note that, here, the sealed portion means a portion in which a plurality of sheets forming the continuous web are connected to each other.

In the manufacturing apparatus according to the fifth aspect, the sealed portion, which is relatively easily detected, is used as a reference for detecting the positional shift of the portion to be cut (to-be-cut portion). Thus, it is possible to accurately detect the positional shift of the portion to be cut (to-be-cut portion) of the continuous web at an imaging location, and therefore, it is possible to accurately control the portion to be cut of the continuous web.

An apparatus for manufacturing an absorbent article according to a sixth aspect is the apparatus for manufacturing an absorbent article according to any one of the first to fifth aspects, in which the second drum includes a plurality of anvils arranged in a circumferential direction of the second drum. The cutting device includes a cutting member. The cutting device is configured to cut the continuous web by sandwiching the continuous web between the cutting member and each of the plurality of anvils of the second drum. A width of each of the plurality of anvils in the circumferential direction of the second drum is wider than a width between a pair of sealed portions provided in the continuous web and on opposite sides of the portion to be cut.

In the manufacturing apparatus according to the sixth aspect, by setting the width of the anvil to be wider than the width of the pair of sealed portions provided in the continuous web and on opposite sides of the portion to be cut (to-be-cut portion), it is possible to perform the cutting while sandwiching the to-be-cut portion between the cutting member and the anvil even when the cutting timing of the cutting device is slightly shifted in accordance with the positional shift.

An apparatus for manufacturing an absorbent article according to a seventh aspect is the apparatus for manufacturing an absorbent article according to the sixth aspect, in which the second drum includes support portions provided on both sides of each of the plurality of anvils in the circumferential direction of the second drum, the support portions being configured to support the continuous web.

In the manufacturing apparatus according to the seventh aspect, the continuous web is supported by the support portions provided on both sides of the anvil in the circumferential direction of the second drum. Thus, it is possible to reduce the occurrence of a problem such as a positional shift due to the occurrence of wrinkles in the continuous web at the time of cutting.

An apparatus for manufacturing an absorbent article according to an eighth aspect is the apparatus for manufacturing an absorbent article according to the first to seventh aspects, further including a discharge device and a second controller. The discharge device is disposed downstream of the second drum, in the conveyance direction of the continuous web, and is configured to discharge the absorbent article to an outside. The second controller is configured to control the operation of the discharge device. The second controller is configured to cause, by controlling the operation of the discharge device, the absorbent article that is defective to be discharged to the outside.

In the manufacturing apparatus according to the eighth aspect, even when the continuous web is not cut at an appropriate position, it is possible to automatically discharge a generated defective absorbent article to the outside.

An apparatus for manufacturing an absorbent article according to a ninth aspect is the apparatus for manufacturing an absorbent article according to the first to eighth aspects, in which the first drum is configured to change tension applied in the conveyance direction of the continuous web, during conveyance of the continuous web. The detection device is configured to detect the portion to be cut of the continuous web after the tension has been changed in the first drum.

In the manufacturing apparatus according to the ninth aspect, by performing tension adjustment in the first drum, the absorbent articles having different sizes can be manufactured by one apparatus, without performing complicated adjustment or the like. On the other hand, in a case where there are no measures, the continuous web stretches and contracts with respect to a desired length when tension adjustment is performed, and in the cutting step for the continuous web, there is a possibility that the continuous web is cut at a position shifted from the to-be-cut portion and that a desired product may not be obtained. However, here, the detection device is provided, whereby it is possible to detect the positional shift of the to-be-cut portion at the detection location or the positional shift of the portion to be actually cut by the cutting device. Thus, it is possible to detect the occurrence of such a problem, and reduce the occurrence of such a problem, for example.

A method for manufacturing an absorbent article according to a tenth aspect includes a first conveyance step, a second conveyance step, a cutting step, and a detection step. In the first conveyance step, a continuous web is conveyed by a first drum. In the second conveyance step, the continuous web conveyed by the first drum is further conveyed by a second drum. In the cutting step, a predetermined portion of the continuous web is cut by a cutting device disposed to face the second drum to form an absorbent article. In the detection step, a portion to be cut of the continuous web that the first drum or the second drum conveys is detected by a detection device, on an upstream side of a cutting location at which the continuous web is to be cut by the cutting device in a conveyance direction of the continuous web.

In the manufacturing method according to the tenth aspect, it is possible to detect a positional shift of the portion to be cut (a positional shift of a to-be-cut portion at a detection location or a positional shift of a portion to be actually cut by the cutting device) at an early stage, based on a detection result of the detection device.

A method for manufacturing an absorbent article according to an eleventh aspect is the method for manufacturing an absorbent article according to the tenth aspect, further including an imaging step of imaging, by an imaging device, the continuous web. In the detection step, the portion to be cut of the continuous web is detected, based on an image imaged by the imaging device.

In the manufacturing apparatus according to the eleventh aspect, it is possible to detect the positional shift of the portion to be cut at an early stage, based on the image.

A method for manufacturing an absorbent article according to a twelfth aspect is the method for manufacturing an absorbent article according to the eleventh aspect, in which, in the imaging step, the imaging device images a pair of sealed portions provided in the continuous web and on opposite sides of the portion to be cut, in the conveyance direction of the continuous web. In the cutting step, a first controller controls an operation of the cutting device, based on a timing at which the pair of sealed portions are imaged.

Note that, here, the sealed portion means a portion in which a plurality of sheets forming the continuous web are connected to each other.

In the manufacturing method according to the twelfth aspect, the positional shift of the portion to be cut (to-be-cut portion) of the continuous web at an imaging location is detected based on the image imaged by the imaging device, based on which the operation of the cutting device is controlled. Thus, it is possible to reduce the positional shift of the portion to be cut of the continuous web.

In particular, here, the sealed portion, which is relatively easily detected, is used as a reference for detecting the positional shift of the portion to be cut (to-be-cut portion). Thus, it is possible to accurately detect the positional shift of the portion to be cut (to-be-cut portion) of the continuous web at the imaging location, and therefore, it is possible to accurately control the portion to be cut of the continuous web.

A method for manufacturing an absorbent article according to a thirteenth aspect is the method for manufacturing an absorbent article according to any one of the tenth to twelfth aspects, further including a discharge step. In the discharge step, a second controller operates a discharge device disposed downstream of the second drum in the conveyance direction of the continuous web to cause the absorbent article that is defective to be discharged to an outside.

In the manufacturing method according to the thirteenth aspect, even when the continuous web is not cut at an appropriate position, it is possible to automatically discharge a generated defective absorbent article to the outside.

A method for manufacturing an absorbent article according to a fourteenth aspect is the method for manufacturing an absorbent article according to any one of the tenth to thirteenth aspects, in which, in the first conveyance step, tension applied in the conveyance direction of the continuous web is changed during conveyance of the continuous web. In the detection step, the portion to be cut of the continuous web after the tension has been changed in the first conveyance step is detected.

In the manufacturing method according to the fourteenth aspect, since tension adjustment is performed in the first drum, the absorbent articles having different sizes can be manufactured by one apparatus, without performing complicated adjustment or the like. On the other hand, in a case where there are no measures, the continuous web stretches and contracts with respect to a desired length when tension adjustment is performed, and in the cutting step for the continuous web, there is a possibility that the continuous web is cut at a position shifted from the to-be-cut portion and that a desired product may not be obtained. However, here, the detection device is provided, whereby it is possible to detect the positional shift of the to-be-cut portion at the detection location or the positional shift of the portion to be actually cut by the cutting device. Thus, it is possible to detect the occurrence of such a problem, and reduce the occurrence of such a problem, for example.

### Reference Signs List

- 1: Diaper (absorbent article)
- 5: Side sealed portion (sealed portion)
- 200: First drum (first drum)
- 300: Second drum (second drum)
- 320: Secondary suction member (support portion)
- 330: Anvil
- 400: Imaging device
- 500: Cutting device
- 510: Cutting member
- 600: Discharge device
- 700: Control device (detection device, first controller, second controller)
- C: Continuous web
- E: Width between side sealed portions (width between sealed portions)
- L: Width of anvil
- Y: To-be-cut portion (portion to be cut)

## Claims

1. An apparatus for manufacturing an absorbent article, comprising:
a first drum configured to convey a continuous web;
a second drum configured to receive the continuous web conveyed by the first drum and further convey the continuous web;
a cutting device disposed to face the second drum, the cutting device being configured to cut a predetermined portion of the continuous web to form an absorbent article; and
a detection device configured to detect, on an upstream side of a cutting location at which the continuous web is to be cut by the cutting device in a conveyance direction of the continuous web, a portion to be cut of the continuous web that the first drum or the second drum conveys.

2. The apparatus for manufacturing an absorbent article according to claim 1, further comprising an imaging device configured to image the continuous web, wherein
the detection device is configured to detect the portion to be cut of the continuous web, based on an image imaged by the imaging device.

3. The apparatus for manufacturing an absorbent article according to claim 2, wherein the imaging device is configured to image at least the portion to be cut of the continuous web by the cutting device.

4. The apparatus for manufacturing an absorbent article according to any one of claims 1 to 3, further comprising a first controller configured to control an operation of the cutting device, based on a detection result of the detection device.

5. The apparatus for manufacturing an absorbent article according to claim 2 or 3, further comprising a first controller configured to control an operation of the cutting device, based on a detection result of the detection device, wherein
the imaging device is configured to image a pair of sealed portions provided in the continuous web and on opposite sides of the portion to be cut of the continuous web by the cutting device, in the conveyance direction of the continuous web, and
the first controller is configured to control the operation of the cutting device, based on a timing at which the pair of sealed portions are imaged.

6. The apparatus for manufacturing an absorbent article according to any one of claims 1 to 5, wherein
the second drum includes a plurality of anvils arranged in a circumferential direction of the second drum,
the cutting device includes a cutting member, the cutting device being configured to cut the continuous web by sandwiching the continuous web between the cutting member and each of the plurality of anvils of the second drum, and
a width of each of the plurality of anvils in the circumferential direction of the second drum is wider than a width between a pair of sealed portions provided in the continuous web and on opposite sides of the portion to be cut.

7. The apparatus for manufacturing an absorbent article according to claim 6, wherein the second drum includes support portions provided on both sides of each of the plurality of anvils in the circumferential direction of the second drum, the support portions being configured to support the continuous web.

8. The apparatus for manufacturing an absorbent article according to any one of claims 1 to 7, further comprising:
a discharge device disposed downstream of the second drum, in the conveyance direction of the absorbent article, the discharge device being configured to discharge the absorbent article to an outside; and
a second controller configured to control an operation of the discharge device, wherein
the second controller is configured to cause, by controlling the operation of the discharge device, the absorbent article that is defective to be discharged to the outside.

9. The apparatus for manufacturing an absorbent article according to any one of claims 1 to 8, wherein
the first drum is configured to change tension applied in the conveyance direction of the continuous web, during conveyance of the continuous web, and
the detection device is configured to detect the portion to be cut of the continuous web after the tension has been changed in the first drum.

10. A method for manufacturing an absorbent article, comprising:
a first conveyance step of conveying, by a first drum, a continuous web;
a second conveyance step of further conveying, by a second drum, the continuous web conveyed by the first drum;
a cutting step of cutting, by a cutting device disposed to face the second drum, a predetermined portion of the continuous web to form an absorbent article; and
a detection step of detecting, by a detection device, a portion to be cut of the continuous web that the first drum or the second drum conveys, on an upstream side of a cutting location at which the continuous web is to be cut by the cutting device in a conveyance direction of the continuous web.

11. The method for manufacturing an absorbent article according to claim 10, further comprising an imaging step of imaging, by an imaging device, the continuous web, wherein
in the detection step, the portion to be cut of the continuous web is detected, based on an image imaged by the imaging device.

12. The method for manufacturing an absorbent article according to claim 11, wherein
in the imaging step, the imaging device images a pair of sealed portions provided in the continuous web and on opposite sides of the portion to be cut, in the conveyance direction of the continuous web, and
in the cutting step, a first controller controls an operation of the cutting device, based on a timing at which the pair of sealed portions are imaged.

13. The method for manufacturing an absorbent article according to any one of claims 10 to 12, further comprising a discharge step of discharging, by a second controller operating a discharge device disposed downstream of the second drum in the conveyance direction of the continuous web, the absorbent article that is defective to an outside.

14. The method for manufacturing an absorbent article according to any one of claims 10 to 13, wherein
in the first conveyance step, tension applied in the conveyance direction of the continuous web is changed during conveyance of the continuous web, and
in the detection step, the portion to be cut of the continuous web after the tension has been changed in the first conveyance step is detected.
